# FASCICULE DE BREVET EUROPEEN

(11) **EP 1 710 259 B1**
(45) Date de publication et mention de la délivrance du brevet: **18.04.2018**
(21) Numéro de dépôt: 06300353.7
(22) Date de dépôt: 14.01.1999
(51) Int. Cl.: A61K 8/06, A61K 8/81, C08F 2/32, C08F 220/58, C08F 220/06, A61Q 19/00, A61Q 17/04, A61Q 5/00, A61Q 1/00, A61Q 19/04, A61Q 19/06, A61Q 1/02, A61Q 1/14

(54) **Composition cosmétique épaissie avec un latex inverse**
Mit einem inversen Latex verdickte kosmetische Zusammensetzung
Cosmetic composition thickened with an inverse latex

(30) Priorité: 16.01.1998 FR 9800464; 10.02.1998 FR 9801525; 04.08.1998 FR 9809999
(43) Date de publication de la demande: 11.10.2006
(62) Demande divisionnaire de: 99900919.4
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: MALLO, PAUL, 78290, CROISSY- SUR- SEINE (FR); TABACCHI, GUY, 75007, PARIS (FR); BOITEUX, JEAN-PIERRE, 81710, SAIX (FR)
(74) Mandataire: Conan, Philippe Claude

(56) Documents cités:
- EP-A1- 0 645 429
- EP-A2- 0 503 853
- EP-B1- 1 369 435
- WO-A-92/21318
- WO-A-99/36445
- JP-A- 9 157 130
- US-A- 4 358 355
- US-A- 4 404 051

## Description

La présente demande concerne l'utilisation de latex eau dans huile épaississants pour la fabrication de préparations topiques cosmétiques. Différents épaississants existent et sont déjà utilisés pour ces usages. On connaît en particulier les produits naturels tels que les gommes de guar ou l'amidon mais dont les inconvénients sont ceux inhérents aux produits naturels, tels que la fluctuation des cours, les difficultés d'approvisionnement et une qualité aléatoire.

Les polymères synthétiques sous forme de poudre, principalement les polyacides acryliques sont également largement utilisés mais présentent l'inconvénient de nécessiter une neutralisation lors de l'utilisation, car ils ne développent leur viscosité qu'à partir d'un pH > 6.5 et leur mise en solution est souvent fastidieuse. La demande de brevet japonais publiée sous le numéro 157,130/1997, divulgue des copolymères épaississants acide acrylique / acrylamido 2-méthyle 2-propane sulfonate de sodium qui comportent au moins 45% molaire de monomères acrylique et stables en milieu acide et en présence de sels. Il existe aussi des polymères épaississants synthétiques, se présentant sous forme de latex inverse, c'est-à-dire dont la phase continue est une huile. La mise en solution de ces latex est extrêmement rapide ; les polymères contenus dans ces latex inverses, sont le plus souvent des copolymères acrylamide / acrylate de métal alcalin ou acrylamide/acrylamido 2-méthyl 2-propane sulfonate de sodium ; ils sont déjà neutralisés et lorsqu'ils sont mis en solution dans l'eau, par exemple à une concentration de 1%, on observe que le pH est généralement supérieur à 6.

Les copolymères acrylamide/acrylate de sodium ne développent cependant pas de propriétés épaississantes importantes lorsqu'on abaisse le pH en dessous de 6 ; par contre les copolymères acrylamide/acrylamido 2-méthyl 2-propane sulfonate de sodium décrits dans EP 0 503 853, gardent une capacité épaississante importante même à pH 4.

Cependant, de tels copolymères présentent des teneurs en monoacrylamide qui, bien qu'extrêmement faibles, pourraient conduire à rendre leur utilisation en cosmétique impossible dans un futur proche, suite à l'évolution de la législation européenne sur les substances dangereuses.

La demanderesse s'est donc intéressée à la synthèse et à la mise au point de polymères épaississants, même en pH acide, sous forme de latex inverse sans utiliser de mono-acrylamide.

L'invention a pour objet une composition topique cosmétique, caractérisée en ce qu'elle comprend de 0,1 % à 10 % en poids d'un latex inverse comprenant une phase huile, une phase aqueuse, au moins un agent émulsifiant de type eau dans huile (E/H), au moins un agent émulsifiant de type huile dans eau (H/E), de 20 % à 60 % en poids, et de préférence de 30% à 45% en poids, d'un polyélectrolyte anionique, branché ou réticulé, à base d'un acide 2-méthyl 2-[(1-oxo 2-propènyl) amino] 1-propane-sulfonique partiellement ou totalement salifié sous forme de sel de sodium ou de sel d'ammonium copolymérisé avec l'acide acrylique, partiellement salifié sous forme de sel de sodium ou de sel d'ammonium, 30 % à 90 % des motifs monomèriques que le polyélectrolyte anionique comprend, étant l'acide 2-méthyl 2-[(1-oxo 2-propènyl) amino] 1-propane-sulfonique partiellement ou totalement salifiée sous forme de sel de sodium ou de sel d'ammonium, ledit polyélectrolyte anionique, branché ou réticulé, étant préparé sans utiliser de mono-acrylamide, et un principe actif, choisi parmi un agent hydratant, un agent bronzant, un filtre solaire, un antirides, un agent à visée amincissante, un agent antiradicalaire, un agent antiacnéique ou un antifongique. et en ce qu'elle consiste en une émulsion topique comprenant au moins une phase aqueuse et au moins une phase huile. Par " agent émulsifiant du type eau dans huile ", on désigne des agents émulsifiants possédant une valeur de HLB suffisamment faible pour fournir des émulsions eau dans huile tels que les polymères tensioactifs commercialisés sous le nom de HYPERMER™ ou tels que les esters de sorbitan, comme le monooléate de sorbitan commercialisé par la Société SEPPIC sous le nom de marque MONTANE 80™, ou l'isostéarate de sorbitan commercialisé par SEPPIC sous le nom de MONTANE 70™.

Par " agent émulsifiant du type huile dans eau ", on désigne des agents émulsifiants possédant une valeur de HLB suffisamment élevée pour fournir des émulsions huile dans l'eau tels que les esters de sorbitan éthoxylés comme l'oléate de sorbitan éthoxylé avec 20 moles d'oxyde d'éthylène , commercialisé par la société SEPPIC sous le nom de MONTANOX™80.

Par polymère branché, on désigne un polymère non linéaire qui possède des chaînes pendantes de manière à obtenir, lorsque ce polymère est mis en solution dans l'eau, un fort état d'enchevêtrement conduisant à des viscosités à bas gradient très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique. Le polyélectrolyte anionique utilisé comporte des motifs réticulés et/ou des motifs branchés.

L'invention a notamment pour objet une composition telle que définie précédemment, caractérisée en ce que ledit polyélectrolyte anionique est le résultat d'une copolymérisation de ses monomères précurseurs effectuée à un pH inférieur à 4.

L'invention a plus particulièrement pour objet une composition telle que définie précédemment, caractérisée en ce que le polyélectrolyte anionique est réticulé et/ou branché avec un composé diéthylènique ou polyéthylènique dans la proportion molaire exprimée par rapport aux monomères mis en oeuvre, de 0,005% à 1%, et de préférence de 0,01 % à 0,2 % et, plus particulièrement de 0,01 % à 0,1 %, de préférence celle pour laquelle l'agent de réticulation et/ou l'agent de ramification est choisi parmi le diméthacrylate d'éthylèneglycol, le diallyloxyacétate de sodium, le diacrylate d'éthylèneglycol, le diallyl urée, le triméthylol propanetriacrylate ou le méthylène-bis-(acrylamide).

Le latex inverse contient généralement de 2,5% à 15% en poids, et de préférence de 4% à 9% en poids, d'agents émulsifiants, parmi lesquels de 20% à 50%, notamment de 25% à 40% du poids total des agents émulsifiants présents, sont du type eau dans huile (E/H) et dans laquelle de 80% à 50%, notamment de 75% à 60%, du poids total des agents émulsifiants, sont du type huile dans eau (H/E).

Selon un aspect particulier, le latex inverse est caractérisé en ce que la phase huile représente de 15% à 40%, de préférence de 20% à 25%, de son poids total.

Cette phase huile est constituée soit par une huile minérale commerciale contenant des hydrocarbures saturés comme les paraffines, les isoparaffines, les cycloparaffines, présentant à température ambiante, une densité entre 0.7 et 0.9 et un point d'ébullition supérieur à 180°C, telle que par exemple l'EXXSOL™ D 100 S, ou le MARCOL™52 commercialisés par EXXON CHEMICAL, l'isohexadécane ou l'isododécane, soit par une huile végétale, soit une huile de synthèse, soit par un mélange de plusieurs de ces huiles.

Selon un aspect préféré de la présente invention, la phase huile est constituée de MARCOL™ 52 ou d'isohexadécane ; l'isohexadécane, qui est identifié dans Chemical Abstracts par le numéro RN = 93685-80-4, est un mélange d'isoparaffines en C₁₂, C₁₆ et C₂₀ contenant au moins 97 % d'isoparaffines en C₁₆, parmi lesquelles le constituant principal est le 2,2,4,4,6,8,8-heptaméthyl nonane (RN = 4390-04-9). Il est commercialisé en France par la société BAYER. Le MARCOL™ 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993).

Les latex contiennent entre 20 % et 50 % d'eau. Les latex utilisés peuvent également contenir divers additifs tels que des agents complexants, des agents de transfert, ou des agents limiteurs de chaîne. Le procédé de préparation du latex inverse est caractérisé en ce que :
a) l'on émulsionne une solution aqueuse contenant les monomères et les éventuels additifs, dans une phase huile en présence d'un ou plusieurs agents émulsifiants de type eau dans huile,
b) l'on amorce la réaction de polymérisation par introduction dans l'émulsion formée en a), d'un initiateur de radicaux libres puis on la laisse se dérouler,
c) lorsque la réaction de polymérisation est terminée, on introduit un ou plusieurs agents émulsifiants de type huile dans eau à une température inférieure à 50°C.

Selon une variante de ce procédé, le milieu réactionnel issu de l'étape b), est concentré par distillation, avant la mise en oeuvre de l'étape c).

Selon une mise en oeuvre préférée du procédé tel que défini précédemment, la réaction de polymérisation est amorcée par un couple oxydo-réducteur, tel que le couple hydroperoxyde de cumène - métabisulfite de sodium, à une température inférieure ou égale à 10°C, puis conduite soit de manière quasi-adiabatique jusqu'à une température supérieure ou égale à 40°C, plus particulièrement supérieure ou égale à 50°C, soit en contrôlant l'évolution de la température.

Selon une autre mise en oeuvre préférée du procédé, la solution aqueuse de départ est ajustée à un pH inférieur ou égal à 4 avant la mise en oeuvre de l'étape c).

L'invention a pour objet une composition topique cosmétique. Une composition topique selon l'invention, destinée à être appliquée sur la peau ou les muqueuses de l'homme ou de l'animal, consiste en une émulsion topique comprenant au moins une phase aqueuse et au moins une phase huile. Cette émulsion topique peut être du type huile dans eau. Plus particulièrement, cette émulsion topique peut consister en une émulsion fluide, telle un lait ou un gel fluide. La phase huile de l'émulsion topique peut consister en un mélange d'une ou plusieurs huiles.

Une composition topique selon l'invention est destinée à une utilisation cosmétique La composition topique est utilisée en tant que composition cosmétique destinée à être appliquée sur la peau ou les muqueuses et comporte un principe actif choisi parmi un agent hydratant, un agent bronzant, un filtre solaire, un antirides, un agent à visée amincissante, un agent antiradicalaire, un agent antiacnéique ou un antifongique.

Une composition topique selon l'invention comporte entre 0,1 % et 10 % en poids de l'agent épaississant défini ci-dessus. Le pH de la composition topique cosmétique est de préférence supérieur ou égal à 5.

La composition topique cosmétique peut en outre comporter des composés classiquement compris dans ce type de compositions, par exemple des parfums, des conservateurs, des colorants, des émollients ou des tensioactifs.

La composition du latex inverse utilisé est un substitut intéressant à celles vendues sous le nom SEPIGEL ™ 305 ou SEPIGEL ™ 501 par la demanderesse, car elle présente aussi une bonne compatibilité avec les autres excipients utilisés pour la préparation de formulations telles que les laits, les lotions, les crèmes, les savons, les bains, les baumes, les shampooings ou les après-shampooings. Elle peut aussi être mise en oeuvre avec lesdits SEPIGEL.

Elle est notamment compatible avec les concentrés décrits et revendiqués dans les publications internationales WO 92/06778, WO 95/04592, WO 95/13863 WO 96/37285, WO 98/22207, WO 98/47610 ou dans FR 2734 496, avec les agents tensioactifs décrits dans WO 93/08204.

Elle est particulièrement compatible avec le MONTANOV® 68, le MONTANOV™ 82, le MONTANOV ™ 202 ou le SEPIPERL™ N. Elle peut également être utilisée dans des émulsions du type de celles décrites et revendiquées dans EP 0 629 396 et dans les dispersions aqueuses cosmétiquement ou physiologiquement acceptable avec un composé organo polysiloxane choisi, par exemple parmi ceux décrits dans WO 93/05762 ou dans WO 93/21316.

Elle peut également être utilisée pour former des gels aqueux à pH acide cosmétiquement ou physiologiquement acceptables, tels que ceux décrit dans WO 93/07856; elle peut également être utilisée en association avec des celluloses non-ioniques, pour former par exemple des gels de coiffage tels que ceux décrits dans EP 0 684 024, ou encore en association avec des esters d'acides gras et de sucre , pour former des compositions pour le traitement du cheveux ou de la peau telles que celles décrites dans EP 0 603 019, ou encore dans les shampooings ou après-shampooings tels que décrits et revendiqués dans WO 92/21316 ou enfin en association avec un homopolymère anionique tels que le CARBOPOL™ pour former des produits de traitement des cheveux comme ceux décrits dans DE 195 23596.

La composition du latex inverse utilisé est également compatible avec les principe actifs tels que par exemple, les agents auto-bronzants comme le dihydroxy-acétone (DHA) ou les agents anti-acné; elle peut donc être introduite dans des compositions auto-bronzantes comme celles revendiquées dans EP 0 715 845, EP 0 604249, EP 0576188 ou dans WO 93/07902.

Elle est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561, ou WO 98/09611.

Les exemples qui suivent ont pour but d'illustrer la présente invention.

### Exemple 1: Préparation et propriétés du latex inverse

### A] Préparation

**a)** On charge dans un bécher, sous agitation
   - 200 g d'eau permutée
   - 112,1 g d'une solution aqueuse d'hydroxyde de sodium à 48 % (en poids)
   - 278,4 g de l'acide 2-methyl-2[(1-oxo-2 propenyl) amino] 1-propane sulfonique
   - 73,1 g d'acide acrylique
   - 0,18 g de diéthylène triamine pentacétate de sodium
   - 0,182 g de méthylène-bis-acrylamide

Le pH de la phase aqueuse précédemment décrit est ajusté à 3,5 et la quantité de phase aqueuse est complétée jusqu'à concurrence de 682 g par ajout d'eau permutée.

Parallèlement, on prépare une phase organique en introduisant dans un bécher agité successivement :
- 220 g d'isohexadécane
- 25 g de Montane 80 VG (oléate de sorbitan commercialisé par SEPPIC)
- 0,2 g azo-bis-isobutyronitrile

La phase aqueuse est introduite progressivement dans la phase organique puis soumise à une agitation mécanique violente de type ultra-turrax™ commercialisé par IKA.

L'émulsion obtenue est alors transférée dans un réacteur de polymérisation. L'émulsion est soumise à un barbotage d'azote important de manière à éliminer l'oxygène et refroidit à environ 5-6°C.

On introduit alors 5ml d'une solution contenant 0,42 % (en poids) d'hydroperoxyde de cumène dans l'isohexadécane.

Après un temps suffisant pour une bonne homogénéisation de la solution, on introduit alors une solution aqueuse de métabisulfite de sodium (0,2 g dans 100 ml d'eau) à raison de 0,5 ml/minute. L'introduction est réalisée pendant environ 60 minutes.

Pendant cette introduction, on laisse monter la température dans le réacteur de polymérisation jusqu'à la température finale de polymérisation.

On maintient alors le milieu réactionnel pendant environ 90 minutes à cette température.

L'ensemble est refroidi jusqu'à une température d'environ 35°C et on introduit lentement 50 g d'oléate de sorbitan éthoxylé à 20 moles d'oxyde d'éthylène.

On obtient l'émulsion désirée:

### Evaluation des propriétés

+ viscosité 25°C du latex (Brookfield RVT, Mobile 3, vitesse 20): η = 650 mPas
+ viscosité dans l'eau à 2 % de latex (Brookfield RVT Mobile 6, vitesse 20):
   η = 33800 mPas.
   (Brookfield Mobile 6, vitesse 5): η = 74000 mPas.

On constate que le produit final est exempt d'acrylamide.
**b)** En opérant de la même manière qu'au paragraphe a) à partir de :
   - 200 g d'eau permutée
   - 121,8g d'une solution aqueuse d'hydroxyde de sodium à 48% (en poids)
   - 302,66g de l'acide 2-methyl-2[(1-oxo-2 propenyl) amino] 1-propane sulfonique
   - 49,54g d'acide acrylique
   - 0,18g de diéthylène triamine pentacétate de sodium et
   - 0,163g de méthylène-bis-acrylamide.

On obtient l'émulsion désirée qui présente les caractéristiques suivantes :
+ viscosité dans l'eau à 2% de latex
(Brookfield RVT Mobile 6, vitesse 20):
   η = 29000 mPas
   (Brookfield Mobile 6, vitesse 5): η = 66000 mPas.

On constate que le produit final est aussi exempt d'acrylamide.

On constate que le toucher des émulsions obtenues est très particulier à partir de 1% de polymère dans la solution et que cette différence s'accentue avec l'augmentation de la concentration ; il s'agit d'un toucher très frais au début qui fond complètement sur la peau, toucher que l'on ne ressent pas du tout avec les latex de l'état de la technique.

Les exemples suivants mettent en oeuvre indifféremment les émulsions préparées selon l'un des paragraphes A a) à A b) (appelés dans les exemples suivants - composé de l'exemple 1).

### B] Propriétés

### a) Pouvoir " émulsionnant " de phases grasses

Le latex inverse préparé au paragraphe A] b) (composition 1) a été utilisé pour préparer des émulsions avec différents types de corps gras, apolaires ou polaires, d'origine végétale ou synthétique. Les gels crèmes obtenus dans les différents cas sont stables et d'aspect parfaitement homogène. Leur viscosité est consignée dans le tableau suivant :

| Viscosité à 20°C, en mPa.s | Huile utilisée pour la phase grasse du gel-crème (3 % en composition 1 ; phase grasse : 10%) |
|---|---|
| Brookfield LVT 6rpm | eau distillée : 87 % |
| ≈ 80 000 | Huile de jojoba |
| ≈ 100 000 | Huile d'amande douce |
| ≈ 80 000 | Squalane |
| ≈ 100 000 | Diméthicone |
| ≈ 65 000 | Isohexadécane |
| ≈ 100 000 | Isononyl isononanoate |
| ≈ 100 000 | Cetearyl octanoate |
| ≈ 100 000 | Benzoate C₁₂-C₁₅ |
| ≈ 100 000 | Caprylic / capric Triglycéride |
| ≈ 90 000 | Huile de paraffine |

La composition 1 permet donc de disperser et de stabiliser des phases grasses dans un milieu aqueux, par simple dilution sans qu'une étape de neutralisation soit nécessaire.

### b) stabilité à la température

On a préparé un gel crème comprenant 2,5 % de composition 1 et 20 % de cétéaryl octanoate et mesuré la viscosité. Les résultats sont les suivants :

| | Viscosité Brookfield LVT 6rpm (en mPa.s) |
|---|---|
| | (mesuré à Ta) |
| Après 1 jour à 40 °C | ≈ 69 000 |
| Après 7 jours à 40 °C | ≈ 68 000 |
| Après 1 mois à 40°C | ≈ 66 000 |

### c) Influence du pH sur la viscosité

La viscosité du gel crème préparé avec la composition 1 est très stable au pH dans l'intervalle pH = 6 à pH = 9.

### d) Compatibilité avec les solvants

On a mesuré la viscosité (en mPas), des gels à 3 % en composition 1, dans divers solvants cosmétiques à plusieurs concentrations.

Les résultats consignés dans le tableau suivant montrent que la viscosité de ces gels n'est pas affectée par la présence de solvants.

| Solvant | 20 % | 40 % | 60 % |
|---|---|---|---|
| Hexylène glycol | ≈ 100 000 | ≈ 10 000 | 5000 |
| Ethanol | ≈ 100 000 | 100 000 | 40 000 |
| Dipropylèneglycol | ≈ 100 000 | 100 000 | 90 000 |
| Butylèneglycol | ≈ 100 000 | ≈ 100 000 | ≈ 100 000 |
| Propylène glycol | ≈ 100 000 | ≈ 100 000 | ≈ 100 000 |
| glycérine | ≈ 100 000 | ≈ 100 000 | ≈ 100 000 |

### Exemple 2 : Crème de soin

| | |
|---|---|
| Cyclométhicone : | 10% |
| Composé de l'exemple 1 : | 0,8 % |
| MONTANOV™68: | 4,5 % |
| Conservateur : | 0,65 % |
| Lysine : | 0,025 % |
| EDTA (sel disodique) : | 0,05 % |
| Gomme de xanthane : | 0,2 % |
| Glycérine : | 3% |
| Eau : | qsp 100 % |

### Exemple 3 : Crème de soin

| | |
|---|---|
| Cyclométhicone : | 10 % |
| Composé de l'exemple 1 : | 0,8 % |
| MONTANOV™ 68 : | 4,5 % |
| Perfluoropolymethylisopropylethe r : | 0,5 % |
| Conservateur : | 0,65 % |
| Lysine : | 0,025 % |
| EDTA (sel disodique) : | 0,05 % |
| PEMULEN™TR : | 0,2 % |
| Glycérine : | 3 % |
| Eau : | qsp 100 % |

### Exemple 4 : Baume après-rasage

### FORMULE

| | | |
|---|---|---|
| A | Composé de l'exemple 1 : | 1,5 % |
| | Eau : | q.s.p 100 % |
| | | |
| | | |
| B | MICROPEARL™ M 100: | 5,0 % |
| | SEPICIDE™ CI: | 0,50 % |
| | Parfum: | 0,20 % |
| | Ethanol 95°: | 10,0 % |

### MODE OPERATOIRE

Ajouter B dans A.

### Exemple 5 : Emulsion satinée pour le corps

### FORMULE

| | | |
|---|---|---|
| A | SIMULSOL™ 165 : | 5,0 % |
| | LANOL™ 1688 : | 8,50 % |
| | Beurre de Karité : | 2 % |
| | Huile de paraffine : | 6,5 % |
| | LANOL™ 14M : | 3 % |
| | LANOL™ S : | 0,6 % |
| | | |
| B | Eau : | 66,2 % |
| | | |
| C | MICROPEARL™ M 100: | 5 % |
| | | |
| D | Composé de l'exemple 1: | 3 % |
| | | |
| E | SEPICIDE™ CI : | 0,3 % |
| | SEPICIDE™ HB : | 0,5 % |
| | MONTEINE™ CA : | 1 % |
| | Parfum : | 0,20 % |
| | Acétate de vitamine E : | 0,20 % |
| | Sodium pyrolidinone carboxylate: | 1 % (agent hydratant) |

### MODE OPERATOIRE

Ajouter C dans B, émulsionner B dans A à 70°C, puis ajouter D à 60°C puis E à 30°C.

### Exemple 6 : Lait corporel

### FORMULE

| | | |
|---|---|---|
| A | SIMULSOL™ 165: | 5,0 % |
| | LANOL™ 1688: | 12,0 % |
| | LANOL™ 14M: | 2,0 % |
| | Alcool cétylique: | 0,3 % |
| | SCHERCEMOL™ OP: | 3 % |
| | | |
| B | Eau : | q.s.p. 100% |
| | | |
| C | Composé de l'exemple 1 : | 0,35 % |
| | | |
| D | SEPICIDE™ Cl : | 0,2 % |
| | SEPICIDE™ HB : | 0,5 % |
| | Parfum : | 0,20 % |

| | | |
|---|---|---|
| (Le SCHERCEMOL™ OP est un ester émollient à effet non gras) | | |

### MODE OPERATOIRE

Emulsionner B dans A vers 75°C ; ajouter C vers 60°C, puis D vers 30°C.

### Exemple 7 : crème H/E

### FORMULE

| | | |
|---|---|---|
| A | SIMULSOL™165: | 5,0% |
| | LANOL™1688: 1,0% (additif à effet stabilisant) | 20,0% |
| B | Eau : | q.s.p. 100% |
| C | Composé de l'exemple 1 : | 2,50% |
| | | |
| D | SEPICIDE™ Cl : | 0,20% |
| | SEPICIDE™ HB : | 0,30% |

### MODE OPERATOIRE

Introduire B dans A vers 75°C ; ajouter C vers 60°C, puis D vers 45°C.

### Exemple 8 : gel solaire non gras

### FORMULE

| | | |
|---|---|---|
| A | Composé de l'exemple 1 : | 3,00% |
| | Eau : | 30% |
| B | SEPICIDE™ C : | 0,20% |
| | SEPICIDE™ HB : | 0,30% |
| | Parfum: | 0,10% |
| | | |
| C | Colorant : | q.s. |
| | Eau : | 30% |
| | | |
| D | MICROPEARL™ M 100 : | 3,00% |
| | Eau : | q.s.p 100% |
| | | |
| E | Huile de silicone : | 2,0% |
| | PARSOL™ MCX : | 5,00% |

### MODE OPERATOIRE

Introduire B dans A; ajouter C,puis D, puis E.

### Exemple 9 : Lait solaire

### FORMULE

| | | |
|---|---|---|
| A | SEPIPERL™N : | 3,0% |
| | Huile de sésame : | 5,0% |
| | PARSOL™ MCX : | 5,0% |
| | Carraghénane λ : | 0,10% |
| | | |
| B | Eau : | q.s.p.100% |
| | | |
| C | Composé de l'exemple 1 : | 0,80% |
| | | |
| D | Parfum : | q.s. |
| | Conservateur : | q.s. |

### MODE OPERATOIRE

Emulsionner B dans A à 75°C puis ajouter C vers 60°C, puis D vers 30°C et ajuster le pH si nécessaire

### Exemple 10 : Gel de massage

### FORMULE

| | | |
|---|---|---|
| A | Composé de l'exemple 1: | 3,5% |
| | Eau : | 20,0% |
| | | |
| B | Colorant : | 2 gouttes/100g |
| | Eau : | q.s. |
| | | |
| C | Alcool: | 10% |
| | Menthol : | 0,10% |
| | | |
| D | Huile de silicone : | 5,0% |

### MODE OPERATOIRE

Ajouter B dans A ; puis ajouter au mélange, C puis D.

### Exemple 11 : gel soin de massage

### FORMULE

| | | |
|---|---|---|
| A | Composé de l'exemple 1 : | 3,00% |
| | Eau : | 30% |
| | | |
| B | SEPICIDE™ CI: | 0,20% |
| | SEPICIDE™ HB: | 0,30% |
| | Parfum : | 0,05% |
| | | |
| C | colorant : | q.s. |
| | Eau : | q.s.p 100% |
| D | MICROPEARL™ SQL: | 5,0% |
| | LANOL™ 1688 : | 2% |

### MODE OPERATOIRE

Préparer A; additionner B, puis C, puis D.

### Exemple 12 : Gel coup d'éclat

### FORMULE

| | | |
|---|---|---|
| A | Composé de l'exemple 1: | 4% |
| | Eau : | 30% |
| | | |
| B | ELASTINE HPM: | 5,0% |
| | | |
| C | MICROPEARL™ M 100: | 3% |
| | Eau : | 5% |
| | | |
| D | SEPICIDE™ Cl: | 0,2% |
| | SEPICIDE™ HB: | 0,3% |
| | Parfum: | 0,06% |
| | Sodium pyrolidinone carboxylate 50%: | 1% |
| | Eau : | q.s.p. 100% |

### MODE OPERATOIRE

Préparer A ; additionner B, puis C, puis D.

### Exemple 13 : Lait corporel

### FORMULE

| | | |
|---|---|---|
| A | SEPIPERL™N : | 3,0% |
| | Triheptonate de glycerol : | 10,0% |
| | | |
| B | Eau : | q.s.p.100% |
| | | |
| C | Composé de l'exemple 1: | 1,0% |
| D | Parfum : | q.s. |
| | Conservateur : | q.s. |

### MODE OPERATOIRE

Fondre A à environ 75°C. Emulsionner B dans A à 75°C puis ajouter C vers 60°C, puis D.

### Exemple 14 : Emulsion démaquillante à l'huile d'amande douce

### FORMULE

| | |
|---|---|
| MONTANOV™68 : | 5% |
| Huile d'amandes douces : | 5% |
| Eau : | q.s.p.100% |
| Composé de l'exemple 1: | 0,3% |
| Glycérine : | 5% |
| Conservateur : | 0,2% |
| Parfum : | 03% |

### Exemple 15 : Crème hydratante pour peaux grasses

### FORMULE

| | |
|---|---|
| MONTANOV™68 : | 5% |
| Cétylstéaryloctanoate : | 8% |
| Octyl palmitate : | 2% |
| Eau : | q.s.p.100% |
| Composé de l'exemple 1: | 0,6% |
| MICROPEARL™ M100: | 3,0% |
| Mucopolysaccharides: | 5% |
| SEPICIDE™ HB: | 0,8 |
| Parfum: | 03% |

### Exemple 16 : Baume après-rasage apaisant sans alcool

### FORMULE

| | |
|---|---|
| Mélange de lauryl aminoacides : | 0,1% à 5% |
| Aspartate de magnésium et de potassium : | 0,002% à 0,5% |
| LANOL™ 99 : | 2% |
| Huile d'amandes douces : | 0,5% |
| Eau : | q.s.p.100% |
| Composé de l'exemple 1 : | 3% |
| SEPICIDE™ HB : | 0,3% |
| SEPICIDE™ Cl : | 0,2% |
| Parfum : | 0,4% |

### Exemple 17 : Crème aux AHA pour peaux sensibles

### FORMULE

| | |
|---|---|
| Mélange de lauryl aminoacides : | 0,1% à 5% |
| Aspartate de magnésium et de potassium : | 0,002% à 0,5% |
| LANOL™ 99 : | 2% |
| MONTANOV™ 68 : | 5,0% |
| Eau : | q.s.p.100% |
| Composé de l'exemple 1 : | 1,50% |
| Acide gluconique: | 1,50% |
| Triéthanolamine : | 0,9% |
| SEPICIDE™ HB : | 0,3% |
| SEPICIDE™Cl : | 0,2% |
| Parfum : | 0,4% |

### Exemple 18 : Soin apaisant après-soleil

### FORMULE

| | |
|---|---|
| Mélange de lauryl aminoacides: | 0,1% à 5% |
| Aspartate de magnésium et de potassium: | 0,002% à 0,5% |
| LANOL™ 99: | 10,0% |
| Eau: | q.s.p.100% |
| Composé de l'exemple 1: | 2,50% |
| SEPICIDE™ HB: | 0,3% |
| SEPICIDE™ Cl: | 0,2% |
| Parfum: | 0,4% |
| Colorant: | 0,03% |

### Exemple 19 : Lait démaquillant

### FORMULE

| | |
|---|---|
| SEPIPERL™N | 3% |
| PRIMOL 352: | 8,0% |
| Huile d'amandes douces: | 2% |
| Eau: | q.s.p.100% |
| Composé de l'exemple 1: | 0,8% |
| Conservateur: | 0,2% |

### Exemple 20 : Lait corporel

### FORMULE

| | |
|---|---|
| SEPIPERL™N: | 3,5% |
| LANOL™ 37T: | 8,0% |
| SOLAGUM™L: | 0,05% |
| Eau: | q.s.p.100% |
| Benzophénone: | 2,0% |
| Diméthicone 350cPs: | 0,05% |
| Composé de l'exemple 1: | 0,8% |
| Conservateur: | 0,2% |
| Parfum: | 0,4% |

### Exemple 21: émulsion fluide à pH alcalin

| | |
|---|---|
| MARCOL™ 82: | 5,0% |
| NaOH: | 10,0% |
| Eau: | q.s.p.100% |
| Composé de l'exemple 1: | 1,5% |

### Exemple 22 : Fond de teint fluide

### FORMULE

| | |
|---|---|
| SIMULSOL™ 165 | 5,0% |
| LANOL™ 84D: | 8,0% |
| LANOL™99: | 5,0% |
| Eau: | q.s.p.100% |
| Pigments et charges minérales: | 10,0% |
| Composé de l'exemple 1: | 1,2% |
| Conservateur: | 0,2% |
| Parfum: | 0,4% |

### Exemple 23 : Lait solaire

### FORMULE

| | |
|---|---|
| SEPIPERL™N | 3,5% |
| LANOL™ 37T: | 10,0% |
| PARSOL NOX™: | 5,0% |
| EUSOLEX™ 4360: | 2,0% |
| Eau: | q.s.p. 100% |
| Composé de l'exemple 1: | 1,8% |
| Conservateur: | 0,2% |
| Parfum: | 0,4% |

### Exemple 24 : Gel contour.des yeux

### FORMULE

| | |
|---|---|
| Composé de l'exemple 1: | 2,0% |
| Parfum: | 0,06% |
| Sodium pyrrolidinonecarboxylate: | 0,2% |
| DOW CORNING™ 245 FLuid | 2,0% |
| Eau: | q.s.p. 100% |

### Exemple 25: composition de soin non rincée

### FORMULE

| | |
|---|---|
| Composé de l'exemple 1: | 1,5% |
| Parfum: | q.s |
| Conservateur: | q.s. |
| DOW CORNING™ X2 8360: | 5,0% |
| DOW CORNING™ Q2 1401: | 15,% |
| Eau: | q.s.p. 100% |

### Exemple 26: gel amincissant

| | |
|---|---|
| Composé de l'exemple 1 | 5 % |
| Ethanol | 30 % |
| Menthol | 0,1 % |
| Caféine | 2,5 % |
| Extrait de ruscus | 2 % |
| Extrait de lierre | 2 % |
| SEPICIDE™HB | 1 % |
| Eau | q.s.p. 100 % |

### Exemple 27 : Baume après-rasage apaisant sans alcool

### FORMULE

| | | |
|---|---|---|
| A | LIPACIDE™ PVB : | 1,0% |
| | LANOL™ 99 : | 2,0% |
| | Huile d'amandes douces : | 0,5% |
| | | |
| B | Composé de l'exemple 1 | 3,5% |
| | | |
| C | Eau : | q.s.p. 100% |
| | | |
| D | Parfum: | 0,4% |
| | SEPICIDE™ HB : | 0,4% |
| | SEPICIDE™ Cl : | 0,2% |

### Exemple 28: Gel rafraîchissant après-rasage

### FORMULE

| | | |
|---|---|---|
| A | LIPACIDE™ PVB : | 0,5% |
| | LANOL™ 99 : | 5,0% |
| | Composé de l'exemple 1 | 2,5% |
| | | |
| B | eau : | q.s.p.100% |
| | | |
| C | MICROPEARL™ LM : | 0,5% |
| | Parfum : | 0,2% |
| | SEPICIDE™ HB : | 0,3% |
| | SEPICIDE™ Cl : | 0,2% |

### Exemple 29: Soin pour les peaux grasses

### FORMULE

| | | |
|---|---|---|
| A | MICROPEARL™ M310 : | 1,0% |
| | Composé de l'exemple 1 | 5,0% |
| | Isononanoate d'octyle : | 4.0% |
| | | |
| B | Eau : | q.s.p.100% |
| | | |
| C | SEPICONTROL™ A5 : | 4,0% |
| | Parfum: | 0,1% |
| | SEPICIDE™ HB : | 0,3% |
| | SEPICIDE™ Cl : | 0,2% |
| | | |
| D | CAPIGEL™ 98 : | 0,5% |
| | Eau : | 10% |

### Exemple 30 : Crème aux AHA

### FORMULE

| | | |
|---|---|---|
| A | MONTANOV™ 68 : | 5,0% |
| | LIPACIDE™ PVB : | 1,05% |
| | LANOL™ 99 : | 10,0% |
| B | Eau : | q.s.p. 100% |
| | Acide gluconique : | 1,5% |
| | TEA (triéthanolamine) : | 0,9% |
| | | |
| C | Composé de l'exemple 1 | 1,5% |
| D | Parfum: | 0,4% |
| | SEPICIDE™ HB: | 0,2% |
| | SEPICIDE™ CI : | 0,4% |

### Exemple 31 : Autobronzant non gras pour visage et corps

### FORMULE

| | | |
|---|---|---|
| A | LANOL™ 2681 : | 3,0% |
| | Composé de l'exemple 1 | 2,5% |
| | | |
| B | Eau : | q.s.p.100% |
| | Dihydroxyacétone : | 3,0% |
| | | |
| C | Parfum : | 0,2% |
| | SEPICIDE™ HB : | 0,8% |
| | NaOH (hydroxyde de sodium) : | qs pH = 5 % |

### Exemple 32 : Lait solaire au monoï de Tahiti

### FORMULE

| | | |
|---|---|---|
| A | Monoï de Tahiti : | 10% |
| | LIPACIDE™ PVB : | 0,5% |
| | Composé de l'exemple 1 | 2,2% |
| | | |
| B | Eau : | q.s.p. 100% |
| | | |
| C | Parfum : | 0,1% |
| | SEPICIDE™ HB : | 0,3% |
| | SEPICIDE™ Cl : | 0,1% |
| | Méthoxycinnamate d'octyle : | 4,0% |

### Exemple 33 : Soin solaire pour le visage

### FORMULE

| | | |
|---|---|---|
| A | Cyclométhicone et diméthiconol : | 4,0% |
| | Composé de l'exemple 1 | 3,5% |
| | | |
| B | Eau : | q.s.p. 100% |
| | | |
| C | Parfum: | 0,1% |
| | SEPICIDE™ HB : | 0,3% |
| | SEPICIDE™ Cl : | 0,21% |
| | Méthoxycinnamate d'octyle : | 5,0% |
| | Micatitane : | 2,0% |
| | Acide lactique : | q.s.p. pH = 6,5 |

### Exemple 34 : Emulsion bronzante sans soleil

### FORMULE

| | | |
|---|---|---|
| A | LANOL™ 99 : | 15% |
| | MONTANOV™ 68 : | 5,0% |
| | Paraméthoxycinnamate d'octyle :3,0% | |
| | | |
| B | Eau : | q.s.p. 100% |
| | Dihydroxyacétone : | 5,0% |
| | Phosphate monosodique : | 0,2% |
| | | |
| C | Composé de l'exemple 1 | 0,5% |
| D | Parfum : | 0,3% |
| | SEPICIDE™ HB : | 0,8% |
| | NaOH : | q.s. pH=5 |

### Exemple 35 : Gel brillance

| | |
|---|---|
| Composé de l'exemple 1 | 1,5 % |
| Silicone volatil | 25 % |
| Monopropylèneglycol | 25 % |
| Eau déminéralisée | 10 % |
| Glycérine | qsp 100 % |

### Exemple 36 : Gel amincissant

| | |
|---|---|
| Composé de l'exemple 1 | 1,5 % |
| Isononylisononanoate | 2 % |
| Caféine | 5 % |
| Ethanol | 40 % |
| MICROPEARL™ LM | 2 % |
| Eau déminéralisée | qsp 100 % |
| Conservateur parfum | qs |

### Exemple 37 : Lait démaquillant

| | |
|---|---|
| SIMULSOL™ 165 | 4 % |
| MONTANOV™ 202 | 1 % |
| Caprylate-caprate triglyceride | 15 % |
| PECOSIL™ DCT | 1 % |
| Eau déminéralisée | qs |
| CAPIGEL™ 98 | 0,5 % |
| Composé de l'exemple 1 | 1 % |
| PROTEOL™ OAT | 2 % |
| NaOH | qsp pH 7 |

### Exemple 38 : Crème solaire

| | |
|---|---|
| SIMULSOL™ 165 | 3 % |
| MONTANOV™ 202 | 2 % |
| Benzoate C12-C15 | 8 % |
| PECOSIL™ PS 100 | 2 % |
| Diméthicone | 2 % |
| Cyclométhicone | 5 % |
| Octyl-méthoxy-cinnamate | 6 % |
| Benzophénone-3 | 4 % |
| Oxyde de Titane | 8 % |
| Gomme xanthane | 0,2 % |
| Butylène-glycol | 5 % |
| Eau déminéralisée | qsp 100 % |
| Composé de l'exemple 1 | 1,5 % |
| Conservateur, parfum | qs |

### Exemple 39 : Gel de soin peaux mixtes

| | |
|---|---|
| Composé de l'exemple 1 | 4 % |
| Squalane végétal | 5 % |
| Dimethicone | 1,5 % |
| SEPICONTROL™ A5 | 4 % |
| Gomme xanthane | 0,3 % |
| Eau | qsp 100 % |
| Conservateur, Parfum | qs |

### Exemple 40 : Voile parfumé pour le corps

| | |
|---|---|
| Composé de l'exemple 1 | 1,5 % |
| Cyclométhicone | 5 % |
| Parfum | 2 % |
| MICROPEARL™ M100 | 5 % |
| Glycérine | 5 % |
| Eau déminéralisée | qsp 100 % |

### Exemple 41 : Crème vitaminée

| | |
|---|---|
| SIMULSOL™ 165 | 5 % |
| MONTANOV™ 202 | 1 % |
| Caprylic/capric triglycerides | 20 % |
| Palmitate de vitamine A | 0,2 % |
| Acetate de vitamine E | 1 % |
| MICROPEARL™ M305 | 1,5 % |
| Composé de l'exemple 1 | 0,7 % |
| Eau | qsp 100 % |
| Conservateur, parfum | qs |

Le MONTANOV™ 68 (cétéaryl glucoside), est une composition auto-émulsionnable telle que décrite dans WO 92/06778, commercialisée par la société SEPPIC.

Le MICROPEARL™ M 100 est une poudre ultra fine au toucher très doux et à action matifiante commercialisée par la société MATSUMO

Le SEPICIDE™ Cl, imidazolidine urée, est un agent conservateur commercialisé par la société SEPPIC.

PEMULEN™ TR est un polymère acrylique commercialisé par GOODRICH.

Le SIMULSOL™ 165 est du stéarate de glycérol auto-émulsionnable commercialisée par la société SEPPIC.

Le LANOL™ 1688 est un ester émollient à effet non gras commercialisé par la société SEPPIC.

Le LANOL™ 14M et le LANOL ® S sont des facteurs de consistance commercialisés par la société SEPPIC.

Le SEPICIDE™ HB , qui est un mélange de phénoxyéthanol, de méthyl paraben, d'éthylparaben, de propylparaben et de butylparaben, est un agent conservateur commercialisé par la société SEPPIC.

Le MONTEINE™ CA est un agent hydratant commercialisé par la société SEPPIC.

Le SCHERCEMOL™ OP est un ester émollient à effet non gras.

Le LANOL™ P est un additif à effet stabilisant commercialisé par la société SEPPIC.

Le PARSOL™ MCX est de l'octyl paraméthoxycinnamate; commercialisé par la société GIVAUDAN.

Le SEPIPERL™ N est un agent nacrant, commercialisé par la société SEPPIC, à base d'un mélange d'alkyl poly glucosides tels que ceux décrits dans WO 95/13863.

Le MICROPEARL™ SQL est un mélange de micro particules renfermant du squalane qui se libère sous l'action du massage; il est commercialisé par la société MATSUMO.

Le LANOL™ 99 est de l'isononyl isononanoate commercialisé par la société SEPPIC.

Le LANOL™ 37T est du triheptanoate de glycérol, commercialisé par la société SEPPIC.

Le SOLAGUM™ L est un carraghénane commercialisé par la société SEPPIC.

Le MARCOL™ 82 est une huile de paraffine commercialisée par la société EXXON.

Le LANOL™ 84D est du malate de dioctyle commercialisé par la société SEPPIC.

Le PARSOL NOX™ est un filtre solaire commercialisé par la société GIVAUDAN.

L'EUSOLEX™ 4360 est un filtre solaire commercialisé par la société MERCK.

Le DOW CORNING™ 245 Fluid est de la cyclométhicone, commercialisée par la société DOW CORNING.

Le LIPACIDE™ PVB, est un hydrolysat de protéines de blé acylé commercialisé par la société SEPPIC.

Le MICROPEARL™ LM est un mélange de squalane, de polyméthylméthacrylate et de menthol, commercialisé par la société SEPPIC.

Le SEPICONTROL™ A5 est un mélange capryloy glycine, sarcosine, extrait de cinnamon zylanicum, commercialisé par la société SEPPIC, tel que ceux décrits dans la demande internationale de brevet PCT/FR98/01313 déposée le 23 juin 1998 (WO 99/00109). Le CAPIGEL™ 98 est un copolymère acrylique commercialisé par la société SEPPIC.

Le LANOL™ 2681 est un mélange caprylate, caprate de coprah, commercialisé par la société SEPPIC.

Le MONTANOV™ 202, est une composition APG/alcools gras telle que décrite dans WO 98/47610, commercialisée par la société SEPPIC.

## Revendications

1. Composition topique cosmétique, **caractérisée en ce qu'**elle comprend de 0,1 % à 10 % en poids d'un latex inverse comprenant une phase huile, une phase aqueuse, au moins un agent émulsifiant de type eau dans huile (E/H), au moins un agent émulsifiant de type huile dans eau (H/E), de 20 % à 60 % en poids, et de préférence de 30% à 45% en poids, d'un polyélectrolyte anionique, branché ou réticulé, à base d'un acide 2-méthyl 2-[(1-oxo 2-propènyl) amino] 1-propane-sulfonique partiellement ou totalement salifié sous forme de sel de sodium ou de sel d'ammonium copolymérisé avec l'acide acrylique, partiellement salifié sous forme de sel de sodium ou de sel d'ammonium, 30 % à 90 % des motifs monomèriques que le polyélectrolyte anionique comprend, étant l'acide 2-méthyl 2-[(1-oxo 2-propènyl) amino] 1-propane-sulfonique partiellement ou totalement salifiée sous forme de sel de sodium ou de sel d'ammonium, ledit polyélectrolyte anionique, branché ou réticulé, étant préparé sans utiliser de mono-acrylamide,
et un principe actif, choisi parmi un agent hydratant, un agent bronzant, un filtre solaire, un antirides, un agent à visée amincissante, un agent antiradicalaire, un agent antiacnéique ou un antifongique.
**et en ce qu'**elle consiste en une émulsion topique comprenant au moins une phase aqueuse et au moins une phase huile.

2. Composition topique cosmétique, telle que définie à la revendication 1 **caractérisée en ce qu'**elle consiste en un lait ou un gel fluide.

3. Composition topique cosmétique telle que définie à l'une des revendications 1 ou 2, **caractérisée en ce que** son pH est supérieur ou égal à 5.

## Patentansprüche

1. Topische kosmetische Zusammensetzung, **dadurch gekennzeichnet, dass** sie 0,1 Gew.-% bis 10 Gew.-% eines inversen Latex umfasst, der eine Ölphase, eine wässrige Phase, mindestens einen Emulgator vom Wasser-in-ÖI-Typ (W/O-Typ), mindestens einen Emulgator vom Öl-in-Wasser-Typ (O/W-Typ), 20 Gew.-% bis 60 Gew.-%, und vorzugsweise 30 Gew.-% bis 45 Gew.-% eines verzweigten oder vernetzten anionischen Polyelektrolyts auf Basis von teilweise oder vollständig in die Natriumsalz- oder Ammoniumsalzform überführter 2-Methyl-2-[(1-oxo-2-propenyl)amino]-1-propansulfonsäure, die mit teilweise in die Natriumsalz- oder Ammoniumsalzform überführter Acrylsäure copolymerisiert ist, wobei 30 % bis 90 % der Monomereinheiten, die der anionische Polyelektrolyt umfasst, die teilweise oder vollständig in die Natriumsalz- oder Ammoniumsalzform überführte 2-Methyl-2-[(1-oxo-2-propenyl)amino]-1-propansulfonsäure sind, wobei der verzweigte oder vernetzte anionische Polyelektrolyt ohne Verwendung von Monoacrylamid hergestellt ist,
und einen Wirkstoff umfasst, der aus einem Feuchthaltemittel, einem Bräunungsmittel, einem Sonnenfilter, einem Antifaltenmittel, einem schlankmachenden Mittel, einem Antiradikalmittel, einem Anti-Akne- oder einem Antipilzmittel ausgewählt ist,
**und dadurch, dass** sie in einer topischen Emulsion besteht, die mindestens eine wässrige Phase und mindestens eine Ölphase umfasst.

2. Topische kosmetische Zusammensetzung wie in Anspruch 1 definiert, **dadurch gekennzeichnet, dass** sie in einer Milch oder einem fluiden Gel besteht.

3. Topische kosmetische Zusammensetzung wie in einem der Ansprüche 1 oder 2 definiert, **dadurch gekennzeichnet, dass** ihr pH-Wert größer oder gleich 5 ist.

## Claims

1. Cosmetic topical composition, **characterised in that** it comprises from 0.1% to 10% by weight of an inverted latex comprising an oily phase, an aqueous phase, at least one emulsifier of the water-in-oil (W/O) type, at least one emulsifier of the oil-in-water (O/W) type, from 20% to 60% by weight, and preferably from 30% to 45% by weight, of a branched or crosslinked anionic polyelectrolyte, based on a 2-methyl-2-[(1-oxo-2-propenyl)amino]-1-propane-sulphonic acid partially or totally salified in the form of a sodium salt or ammonium salt copolymerised with acrylic acid, partially salified in the form of sodium salt or ammonium salt, 30% to 90% of the monomer patterns that the anionic polyelectrolyte comprises, being 2-methyl-2-[(1-oxo-2-propenyl)amino]-1-propane-sulphonic acid partially or totally salified in the form of a sodium salt or ammonium salt, said branched or crosslinked anionic polyelectrolyte being prepared without using monoacrylamide,
and an active ingredient, chosen from a moisturiser, a tanning agent, a solar filter, an anti-wrinkle agent, a slimming agent, an anti-radical agent, an anti-acne agent or an antifungal agent.
**and in that** it consists of a topical emulsion comprising at least one aqueous phase and at least one oily phase.

2. Cosmetic topical composition, such as defined in claim 1 **characterised in that** it consists of a milk or fluid gel.

3. Cosmetic topical composition such as defined in one of claims 1 or 2, **characterised in that** its pH is greater than or equal to 5.
